# EUROPEAN PATENT SPECIFICATION

(11) **EP 0 520 962 B1**
(45) Date of publication and mention of the grant of the patent: **02.10.2002**
(21) Application number: 92830330.4
(22) Date of filing: 24.06.1992
(51) Int. Cl.: C12N 15/82, C12N 15/13, A01H 5/00

(54) **Plasmid vectors for gene expression in plants**
Plasmid-Vektoren zur Genexpression in Pflanzen
Vecteurs plasmidiques pour l'expression de gènes dans des plantes

(30) Priority: 28.06.1991 IT RM910474
(43) Date of publication of application: 30.12.1992
(73) Proprietor: Ente per le nuove tecnologie, l'energia e l'ambiente ( ENEA), I-00198 Roma (IT); CONSIGLIO NAZIONALE DELLE RICERCHE, 00185 Roma (IT)
(72) Inventor: Biocca, Silvia c/o Consiglio Naz. delle Ricerche, IT-00159 ROME (IT); Cattaneo, Antonino c/o Consiglio Naz. d. Ricerche, IT-00159 ROME (IT); Benvenuto, Eugenio Dip. Ricerche e Sviluppo, IT-00060 S. Maria di Galeria - ROME (IT); Galeffi, Patrizia Dip. Ricerche e Sviluppo, IT-00060 S. Maria di Galeria - ROME (IT)
(74) Representative: de Simone, Domenico

(56) References cited:
- EP-A- 0 270 248
- WO-A-91/06320
- EMBO JOURNAL vol. 6, 1987, EYNSHAM, OXFORD GB pages 3901 - 3907 JEFFERSON, R.A., ET AL. 'GUS fusions: Beta-glucuronidase as a sensitive and versatile gene fusion marker in higher plants'
- GENE vol. 91, no. 2, 1990, AMSTERDAM NL pages 159 - 166 GEORGES, F., ET AL. 'Design and cloning of a synthetic gene for the flounder antifreeze protein and its expression in plant cells'
- J. CELL. BIOCHEM. SUPPL. vol. 14E, 1990, page 303 LEE, J., ET AL. 'The reduction of the freezing point of tobacco plants transformed with the gene encoding for the antifreeze protein from winter flounder'
- PLANT MOLECULAR BIOLOGY. vol. 17, 1991, DORDRECHT, THE NETHERLANDS. pages 865 - 874 BENVENUTO, E., ET AL. ''Phytoantibodies': a general vector for the expression of immunoglobulin domains in transgenic plants'

## Description

This invention relates to plasmid vectors for gene expression in plants.

More particularly, this invention relates to plasmid vectors that allow exogenous sequences to be expressed in plants, such sequences coding preferably for variable regions of immunoglobulins, such vectors being intended for giving the transformed plant some specific characteristics, and in addition the invention also relates to the process of transforming plants by means of said vectors as well as to the employment of said vectors for giving the transformed plants some new characters.

The numbered list of bibliographic references is reported at the end of this disclosure and the documents are cited with the numerical reference in parentheses ( ).

The employment of bacteria belonging to the genus Agrobacterium for transferring exogenous genes into the nuclear genome of plant cells is already known from the prior art (1, 2).

Among the genes or portions of the same which are potentially usable for being introduced into plant cells there are those coding for the chains of immunoglobulins, because the virtually unlimited inventory of this family of molecules can be widely employed both as a source of specific reagents for biochemical and physiological studies in which it is desired to modulate or to inhibit the action of some definite gene products, and for introducing exogenous molecules in a stable way that are capable of conferring new characters to the receptor plant, as for instance the resistance to chemical products or to pathogenous agents. This is possible because it has been shown that an efficient assembling of antibody chains is obtainable for forming the active molecule, even in non lymphoid cells (3, 4).

Employing the transformation technique which is mediated by Agrobacterium, the production of complete antibody molecules has been obtained in transgenic plants by crossbreeding two plants already transformed with recombinant vectors that expressed respectively gamma- or kappa-immunoglobulin chains (5, 6) or, with one single step, employing as an expression vector a vector comprising two promoters, downstream of which some sequences coding for two different immunoglobulin chains had been sub-cloned (7). Such vectors, and the processes realizable with the same, do not appear to be easily employable, as in the first instance they require long and difficult crossbreeding experiments and in the second instance the vector is not adaptable to the cloning and the expression of immunoglobulins different from those that have been already.

Simpler forms of antibody molecules, recently identified are certainly more suitable for their functional expression in plants, eluding the mentioned assembling difficulties of immunoglobulin chains and of correct transportation of synthesized molecules into the cell compartment wherein the target molecule is present. As is well known, the antibody portion which is capable of reacting with an antigen is represented by the variable domains of the heavy chains (VH) and of the light chains (VL) of immunoglobulins (8). Recently, the cloning and expression of variable domains of the transposed heavy chain (VH) in E.coli has been reported (9), and it has been shown that the variable domain VH alone, without the contribution of the domain VL, is often capable of binding the antigen with a high value of the affinity constant (Patent Application EP 0368684). Such domains, which are called single-domain antibodies (dAbs), represent molecules which are extremely versatile, with respect to the whole antibodies, in experiments of transfer and gene expression.

At last, a chimeric gene has been constructed, comprising sequences coding both for VH chain and for VL chain of a specific antibody; said sequences are conveniently separated by a peptide leading to a correct tridimensional structure of the antibody chains, even they are comprised into a linear polypeptidic chain (sc Fv,10).

Accordingly, there is clearly the need for a plasmid vector for cloning and expressing exogenous genes or portions of them in plants, which vector can be easily and generally employed for genes coding for immunoglobulin chains or portions of immunoglobulin chains, and even for dAbs or scFv.

The authors of the present invention have built a plasmid vector satisfying such requirements, as it is easily employable for cloning any exogenous gene, or a portion of the same, said vector, when introduced into plants, allowing a stable and efficient transcription to be realized. The vector is particularly suitable for introducing genes which code for immunoglobulin chains or for portions of the same, which genes are obtained by the amplification of genes for hybridoma line immunoglobulins, as well as for dAbs or scFv.

Accordingly it is an object of the instant invention a plasmid vector for cloning and expressing portions of immunoglobulin chains, said portions being single-domain antibodies (dAbs) or single chain antibodies (scFv) in plants, comprising at least: a sequence coding for a selectable marker in plants; a sequence capable of promoting the efficient and correct transcription (the promoter sequence) of said portions of immunoglobulin chains in plants; a Pst I restriction site at the 5' position and a Bste II restriction site at 3' position for the unidirectional insertion of said portions of immunoglobulin chains; a detector sequence coding for a detectable marker at the 3' of said restriction sites in the same coding reading frame of said portions of immunoglobulin chains; a sequence capable of promoting the efficient and correct termination of transcription (the terminator sequence) of said portions of immunoglobulin chains in plants.

Preferably the selectable marker is a protein having a phosphotransferase II neomycin activity (NPTII) that confers resistance to plant grown in a culture medium containing Kanamycin.

Preferably the promoter comprises the regulative elements of the 35S CaMV virus gene (the cauliflower mosaic virus).

Preferably the detector sequence codes for a peptide which is capable of reacting with a known antibody.

Preferably the terminator is the terminator of the nopaline synthetase gene.

Preferably the portions of immunoglobulin chains react in a specific way with chemical compound or pathogens agents, conferring to the transformed plant a resistance to said chemical compound or said pathogens agents.

Preferably the vector comprises a sequence coding for a signal peptide, which is covalently linked to the NH₂ terminal of the portions of immunoglobulin chains, capable of promoting the transportation and extracellular secretion of said portions of immunoglobulin chains.

Preferably the plasmid vector for cloning and expressing portions of immunoglobulin chains in plants of the invention consists of the plasmid pBG-d-Ab-BIN, which has been deposited, after transforming E.coli with the same, with the NCAIM under the accession number 001144 on 31st May, 1991, in agreement with the Budapest Treaty.

It is an object of the invention a plant transformation process by means of the plasmid vector of the invention comprising the following steps:
- transforming bacteria belonging to the Agrobacterium genus by means of said plasmid vector, to get transformed bacteria;
- infecting the plant to be transformed with said transformed bacteria;
- selecting transgenic plant so obtained by means of said selectable marker that is expressed in plants;
- checking the expression levels of said
- portions of immunoglobulin chains in transgenic plants by means of the product coded by said detector sequence.

It is an object of the invention plants or parts of the same transformed by means of vectors of the invention. Preferably said parts of plants comprise the reproduction material.

It is an object of the invention plants or parts thereof obtainable by means of the process of the invention.

This invention will be disclosed in the following with reference to some application examples of the same which are not limitative of the invention itself, with reference to the following figures wherein:
- Figure 1 represents a scheme illustrating a way to build one of the vectors which are the object of this invention; the restriction sites labelled by an asterisk (*) have been deleted. Bs: BstE II, C: Cla I, Cs: Csp45 I, E: Eco RI, H: Hind III, K; Kpn I, P: Pst I, S: Sac I, Sm: Sma I, Xb: Xba I, Xh: Xho I;

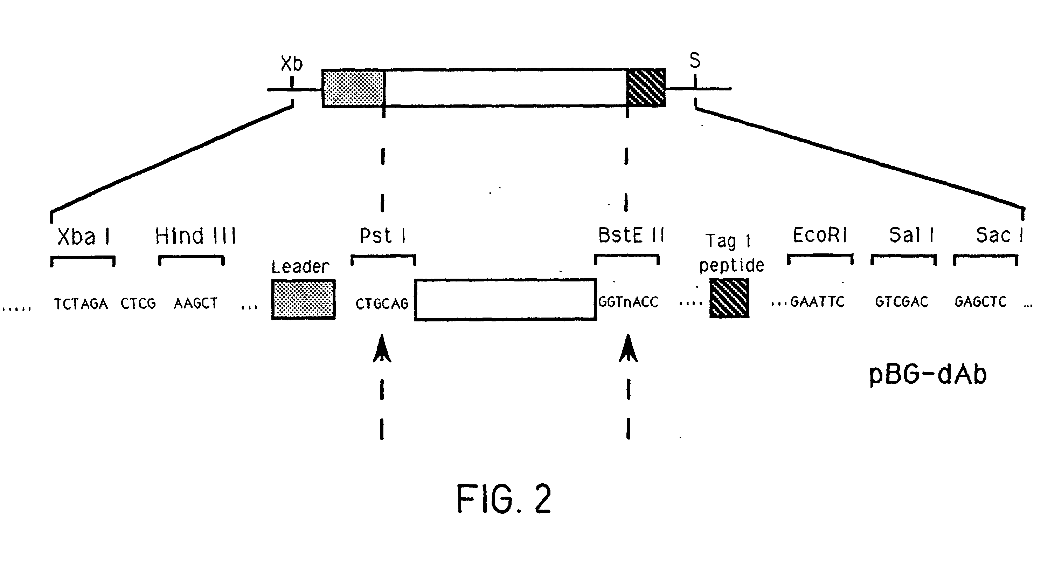

- Figure 2 represents the map of the region that characterizes one of the vectors which are the object of the present invention.

In the examples disclosed in the following, the techniques which are well known to those who are skilled in the art are carried out according to reference (17).

### Example 1

### Constructing the vector pBG-dAb-BIN

The vector pSW1-VHNCI-TAG1 described by (9) has been employed as the starting vector just for exempli≡ fication purposes, but it is to be understood that alternative construction ways are equally possible.

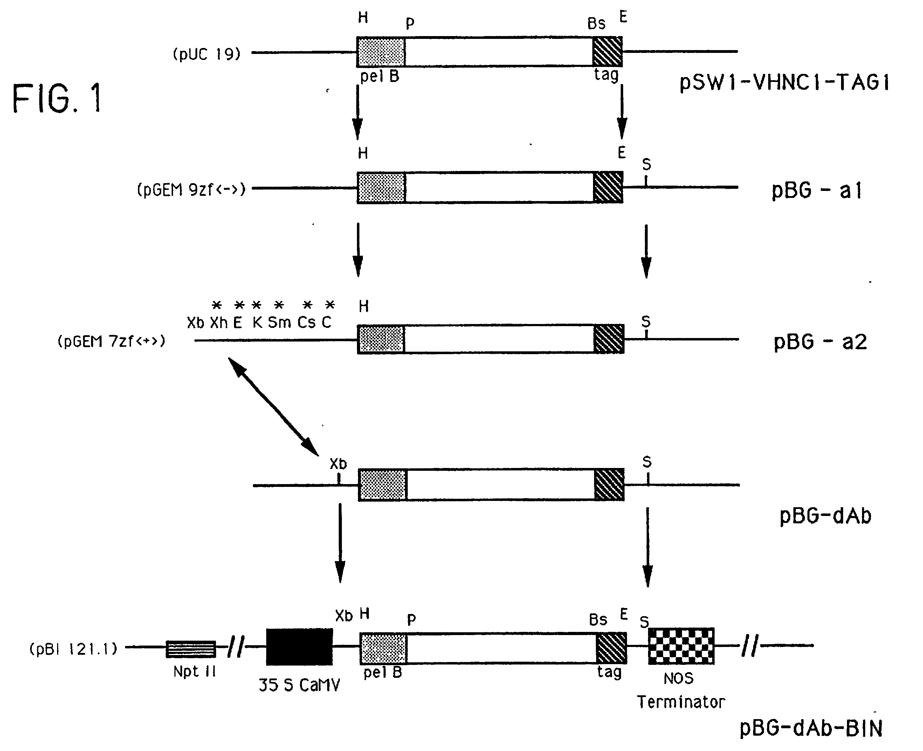

With reference to Figure 1, the fragment Hind III-Eco RI has been excided and subcloned into the sites Hind III-Eco RI of the vector pGEM 7zf(+) (Promega) in which the "polylinker" sites labelled with an asterisk had been removed by means of digestion and treatment with Klenow's fragment of the DNA-polymerase. Following the digestion with Xba I and Sac I, it was possible to subclone the fragment into the vector pB1121 (Clontech Laboratories Inc., Ca).

The removal of the polylinker restriction sites causes the plant active 35S CaMV promoter to be suitably close to the sequence to be transcribed, conferring an optimal in vivo transcription efficiency to the system. By means of the sites Pst I and BstE II, it is possible to insert any exogenous gene into the vector. In particular, as these sites are contained within the primers employed for amplifying from hybridoma lines the sequences that code for immunoglobulin chains (11), such sequences can be easily introduced in said sites without additional steps. The vector so obtained has been called pBG-dAb-BIN and it has been deposited after transforming E.coli with the same and in agreement with the Budapest Treaty with the NCAIM under the accession number 001144, on 31st May, 1991.

### Example 2

### Constructing the vector pBG-NC1-BIN and transforming N.benthamiana with the same

A DNA fragment coding for the heavy chain of the monoclonal antibody NC1/34 HL disclosed in (12) has been introduced into the vector pBG-dAb-BIN just for exemplification purposes, said antibody being capable of recognizing a neuropeptide, i.e. the tachyquinine or substance P, employing the sites PstI and BstE II, so originating the plasmid pBG-NCl-BIN.

Plants of the species N. benthamiana have transformed by means of the plasmid obtained employing techniques which are already known to those who are skilled in the art. Stated briefly, the plasmid pBG-NC1-BIN has been introduced into the strain LBA4404 of A.tumefaciens (13) as disclosed in reference (14) and disks obtained from the leaves of N.benthamiana plants, 4 weeks of age, were infected with the bacteria transformed as disclosed in reference (15). After 3-4 weeks, small plants were evidenced that regenerated on a culture medium suitable to the formation of sprouts (MS, containing 1 mg/ml of 3-indolbutyric acid (IBA), 1 mg/ml of 6benzylaminopurine (BAP) and 30 g/l of sucro≡ se), containing 100 ug/ml of Kanamycin with a 95 % frequency.

Each leaf explantation gave origin to 13.8 ± 1.8 sprouts. The regenerated sprouts were caused to root on a selective medium with 25 ug/ml of Kanamycin containing 1/2 MS with 0.05 mg/ml of IBA and 30 g/ml of sucrose. The plants so obtained showed resistance to Kanamycin through NPTII activity as disclosed in (16). More than 65 % of the plants turned out to be positive.

The putative transgenic plants multiplied in vitro showed normal morphology with respect to the control plants. DNA was extracted from independent plants and hybridized through "Southern" technique employing as probe the fragment Hind III-Eco RI of the pBG-dAb plasmid, putting into evidence a number of 1-5 copies of transformant gene per plant.

### Example 3

### Expression of the NC1/34 antibody in plants: analysis of mRNA and proteins

Cytoplasmic RNA was extracted from the leaves of 15 independent NPTII-positive transformants at the same physiological stage and it was hybridizes through the "Northern" technique employing the same probe as that employed in example 2. Five plants out of those analyzed turned out to be positive, with differences in the expression level not ascribable to the number of copies of the gene or to mRNA degradation phenomena but, quite likely, ascribable to position effects in the plant genome.

Soluble protein extracts were prepared from the leaves of transgenic plants and from the leaves of control plants, and the expression of the protein NC1/34 was tested by means of the "Western" technique, with the monoclonal antibody 9E10 that recognizes the peptide TAG at the C-terminal of the recombinant protein (18). All extracts out of three extracts from transgenic plants showed a protein band which was absent from the control plants. Moreover, the expression levels of the transgenic protein were well correlated with the specific RNA levels in the same plants. The relative amount of the expressed protein varies in the range between 0.1 % and 1 % of the total soluble proteins.

### Example 4

### Locating the protein

The distribution throughout the tissues was investigated by immunofluorescence in sections obtained from leaves, flowers and stems of three transgenic plants, and a strong positive signal was detected in particular in the leaves, the meristems and the flowers.

### BIBLIOGRAPHIC REFERENCES

1. Weising K., Schell J., Kahl G.: Foreign genes in plants: transfer structure, expression, and application. Annu. Rev. Genet. 22: 421-477 (1988).
2. Zambryski P., Tempe J., Schell J.: Transfer and function of T-DNA genes from Agrobacterium Ti and Ri plasmids in Plants. Cell 56: 193-201 (1989).
3. Cattaneo A., Neuberger M.S.: Polymeric immunoglobulin M is secreted by transfectants of non lymphoid cells in the absence of immunoglobulin J chain. EMBO J 6: 2753-2758 (1987).
4. Biocca S., Neuberger M.S., Cattaneo A.: Expression and targeting of intracellular antibodies in mammalian cells. EMBO J 9: 101-108 (1990).
5. Hiatt A., Cafferkey R., Bowdish K.: Production of antibodies in transgenic plants. Nature 342:76-78 (1989).
6. Hiatt A.: Antibodies produced in plants. Nature 344:469470 (1990).
7. During K., Hippe S., Kreuzaler F., Schell J.: Synthesis and self-assembly of a functional monoclonal antibody in trans
   genic Nicotiana tabacum. Plant Mol. Biol. 15: 281-293 (1990).
8. Milstein C.: From the structure of antibodies to the diversification of the immune response. EMBO J 4: 1083-1092 (1985).
9. Ward E.S., Gussow D., Griffiths A.D., Jones P.T., Winter G.: Binding activities of a repertoire of single immunoglobulin variable domains secreted from Escherichia coli. Nature 341: 544-546 (1989).
10. Huston JS et al. Proc Natl Acad Sci USA, 85, 5879-5883 (1988).
11. Orlandi R., Gussow D.H., Jones P.T., Winter G.: Cloning immunoglobulin variable domains for expressione by the polymerase chain reaction. Proc. Natl. Acad. Sci. USA 86: 38333837 (1989).
12. Cuello A.C., Galfre G., Milstein C.: Detection of substance P in the central nervous system by a monoclonal antibody. Proc. Natl. Acad. Sci. USA 76, 3532-3536 (1979).
13. Hoekema A., Hirsch P.R., Hooykaas P.J.J., Schilperoort R.A.: A binary plant vector strategy based on separation of Vir- and T- region of Agrobacterium tumefaciens Ti plasmid. Nature 303: 179-180 (1983).
14. Ditta G., Stanfield S., Corbin D., Helinski D.R.: Broad host range DNA cloning system for Gram negative bacteria: construction of a gene bank of Rhizobium meliloti. Proc. Natl. Acad. Sci. USA 77: 7347-7351.
15. Tavazza R., Ordas R.J., Tavazza M., Ancora G., Benvenuto E.: Genetic transformation of Nicotiana clevelandii using a Ti plasmid derived vector. J. Plant Physiol. 133: 640-644 (1988).
16. Scott R., Draper J., Jefferson R., Dury G., Jacob L.: Analysis of gene organization and expressione in plants. In Draper J. et al. (eds.), Plant Genetic Transformation and Gene Expression, pp. 301-310. Blackwell Scientific Publications, Oxford (1988).
17. Sambrook J. , Fritsch E.f., Maniatis T.: Molecular Cloning. A laboratory manual. II edt. Vol. 1-2-3. CSH Lab. Press (1989).
17. Munro S., Pelham H.: An Hsp70-liceprotein in the ER: identity with the 78 kd glucose-regulated protein and immunoglobulin heavy chain binding protein. Cell 46: 29130 (1986).

## Claims

1. A plasmid vector for cloning and expressing portions of immunoglobulin chains, said portions being single-domain antibodies (dAbs) or single chain antibodies (scFv) in plants, comprising at least: a sequence coding for a selectable marker in plants; a sequence capable of promoting the efficient and correct transcription (the promoter sequence) of said portions of immunoglobulin chains in plants; a Pst I restriction site at the 5' position and a Bste II restriction site at 3' position for the unidirectional insertion of said portions of immunoglobulin chains; a detector sequence coding for a detectable marker at the 3' of said restriction sites in the same coding reading frame of said portions of immunoglobulin chains; a sequence capable of promoting the efficient and correct termination of transcription (the terminator sequence) of said portions of immunoglobulin chains in plants.

2. A plasmid vector for cloning and expressing portions of immunoglobulin chains in plants according to claim 1, wherein said selectable marker is a protein having a phosphotransferase II neomycin activity (NPTII) that confers resistance to plant grown in a culture medium containing Kanamycin.

3. A plasmid vector for cloning and expressing portions of immunoglobulin chains in plants according to any one of the preceding claims wherein said promoter comprises the regulative elements of the 35S CaMV virus gene (the cauliflower mosaic virus).

4. A plasmid vector for cloning and expressing portions of immunoglobulin chains in plants according to any one of the preceding claims wherein said detector sequence codes for a peptide which is capable of reacting with a known antibody.

5. A plasmid vector for cloning and expressing portions of immunoglobulin chains in plants according to any one of the preceding claims wherein said terminator is the terminator of the nopaline synthetase gene.

6. A plasmid vector for cloning and expressing portions of immunoglobulin chains in plants according to any one of the preceding claims wherein said portions of immunoglobulin chains react in a specific way with chemical compound or pathogens agents, conferring to the transformed plant a resistance to said chemical compound or said pathogens agents.

7. A plasmid vector for cloning and expressing portions of immunoglobulin chains in plants according to any one of the preceding claims wherein said vector comprises a sequence coding for a signal peptide, which is covalently linked to the NH₂ terminal of the portions of immunoglobulin chains capable of promoting the transportation and extracellular secretion of said portions of immunoglobulin chains.

8. A plasmid vector for cloning and expressing portions of immunoglobulin chains in plants according to any one of the preceding claims, which consists of the plasmid pBG-d-Ab-BIN, which has been deposited, after transforming E.coli with the same, with the NCAIM under the accession number 001144 on 31st May, 1991, in agreement with the Budapest Treaty.

9. A plant transformation process by means of the plasmid vector according to any one of the preceding claims comprising the following steps:
- transforming bacteria belonging to the Agrobacterium genus by means of said plasmid vector, to get transformed bacteria;
- infecting the plant to be transformed with said transformed bacteria;
- selecting transgenic plant so obtained by means of said selectable marker that is expressed in plants;
- checking the expression levels of said portions of immunoglobulin chains in transgenic plants by means of the product coded by said detector sequence.

10. Plants or parts of the same transformed by means of vectors according to any one of the preceding claims 1-8.

11. Parts of a plant according to claim 10, wherein said parts comprise the reproduction material.

12. Plants or parts thereof according to claim 10 or 11, being obtainable by means of the process according to claim 9.

## Patentansprüche

1. Plasmidvektor zur Klonierung und Expression von Teilen derlmmunoglobulin - Ketten, wobei die genannten Teile Single-Domäne - Antikörper (dAbs) oder Single - Kette-Antikörper (scFV) in Pflanzen sind; enthaltend mindestens eine Sequenz-Kodierung für einen selektivbaren Marker in Pflanzen: eine zur Promotion von wirksamen und korrekten Transkription der vorgenannten Teile von Immunoglobulin -Ketten in Pflanzen geeignete Sequenz (die Promotion - Sequenz): ein Pst I Beschränkrungs ort in der 5. Stelle und ein Bste II Beschränkungsort in der 3.Stelle für ein einsinniges Einfügen der vorgenannte Teile von Immunoglobulin - Ketten; eine Detektor - Sequenze - Kodierung fur einen erfassbaren Marker in der 3.Stelle der vorgenannten Beschränkungsorten im gleichen Kodierung - Lesemahmen der vorgennaten Immunoglobuline - Ketten; eine zur Promotion der wirksamen und korrekten Transkription - Abschlusses (die Abschlussequenz) der vorgenannten Teile von Immunoglobulin in Pflangen geeignete Sequenz.

2. Plasmidvektor zur Klonierung und Expression von Teilen der Immunoglobulin - Ketten in Pflanzen nach Ansprunch 1, worin der selektivbare Marker ein eine Phosphotransferase - II - Neomyein - Aktivität (NPTII) aufweisendes Protein ist, das den in einem Kanamycin enthaltendem Kulturmittel gewachsen Pflanzen eine Widerstandsfähigkeit anverleiht.

3. Plasmidvektor zur Klonierung und Expression von Teilen derlmmunoglobulin - Ketten in Pflanzen nach einem jeden der vorhergehenden Ansprüche, worin der vorgenannte Promotor Regulations elemente des 355 caMV Virus-Gens (der Blumenkohl - Mosaikvirus) enthält.

4. Plasmidvektor zur Klonierung und Expression von Teilen der Immunoglobulin - Ketten in Pflanzen nach einem jeden der vorhergehenden Ansprüche, worin die vorgenannte Detektorsequenz ein Peptid kodiert, das mit einem bekannten Antikörper zu reagieren vermag.

5. Plasmidvektor zur Klonierung und Expression von Teilen derlmmunoglobulin - Ketten in Pflanzen nach einem jeden der vorhergehenden Ansprüche, worin der vorgenannte Terminator der Terminator des Nopaline - Synthetase - Gens ist.

6. Plasmidvektor zur Klonierung und Expression von Teilen der Immunoglobulin - Ketten in Pflanzen nach einem jeden der vorhergehenden Ansprüche, worin die vorgenannten Teile von Immunoglobulin - Ketten in einer spezifischen Weise mit einer chemischen Verbindung oder mit pathogenen Agenzien reagiert, so dass sie der umgeformten Pflanze eine Wiederstandsfähigkeit gegenUber der vorgenannten Venbindung oder den vorgenannten dremischen Agenzien anverleiht wird.

7. Plasmidvektor zur Klonierung und Expression von Teilen der Immunoglobulin - Ketten in Pflanzen nach einem jeden der vorhergehenden Ansprüche, worin der vorgenannte Vektor eine Sequenz - Kode für ein Signal - Peptid enthält, das an das NH₂- Terminal der zur Promotion der Beförderung und extrazellularen Sekretion der vorgenannte Teile von Immuglobuline Ketten geeigneten Teile von Immunoglobulin - Ketten kovalent gebunden ist.

8. Plasmidvektor zur Klonierung und Expression von Teilen der Immunoglobulin - Ketten in Pflanzen nach einem jeden der vorhergehenden Ansprüche, welcher aus dem p-BG-d-Ab-BIN-Plasmid besteht, das nach der E-coli Umformung mit demselben, mit dem NCAIM unter der Zutritt-Nummer 00144 am 31 Mai 1991, gemäss dem Budapest - Abkonmen hinterlegt wurde.

9. Verfahren zur Umformung von Pflanzen vermittels des Plasmidverktors nach je einem der vorkergenden Anprüche, das folgende Verfahrensschritte aufweist:
- Umformung von zum Agrobacterium gehörenden Bakterien vermittels des vorgenannten Plasmidvektor, um umgeformte Bakterien zu erhalten;
- Infizieren der umzuformende Pflanzen mit den vorgenannten umgeformten Bakterien.
- Auswählen einer so erhaltenen transgenischen Pflanze durch den vorgenannten selektierbaren Marker, der in Pflangen vorgesehen ist;
- Überprüfung der Expressions - Niveaus der vorgenannten Teile von Immunoglobulin - Ketten in transgenischen Pflanzen vermittels des durch die vorgenannte Detektor - Sequenz kodierten Produkts.

10. Pflanze oder deren Teile, umgeformt vermittels der Vektore gemäss je einem der vorhergehenden Anspüche 1 bis 8.

11. Teile einer Pflanze gemäs Anspruch 10, worin die vorgenannten Teile das Reproductionsmaterial enthalten.

12. Pflanze oder deren Teile gemäss Anspruch 10 oder 11, welche durch das Verfahren gemäss dem Anspruch 9 enhaltbar sind.

## Revendications

1. Vecteur plasmidique pour la clonage et l'expression de portions des chaînes d'immunoglobulines, lesdites portions étant des anticorps à single domaine (dAbs) ou des anticorps à single chaîne (scFv) dans des plantes, comprenant au moins: une codification des séquences pour un marqueur sélectionnable dans des plantes, une codification des séquences pour promouvoir la transcription efficace et correcte (la séquence des promoteurs) desdites portions des chaînes d'immunoglobulines dans des plants; un site de restriction Pst I à la 5. position et un site de restriction Bste II à la 3. position pour l'insertion unidirectionnel desdites portions des chaînes d'immunoglobulines; une codification de séquences des détecteurs pour un marqueur détectable à la 3. position desdits sites de restriction dans le même cadre de codfication et lecture desdites portions des chaîne d'immunoglobuline; une séquence capable de promouvoir la terminaison efficace et correcte de la transcription (la séquence de terminateurs) desdites portions des chaînes d'immunoglobulines dans des plantes.

2. Vecteur plasmidique pour la clonage et l'expression de portions des chaînes d'immunoglobulines dans des plantes, selon la revendication 1, dans lequel ledit marqueur sélectionable est un protéine ayant une activité de phosphotransferase II néomycine (NPT II) qui confère de résistance à des plantes poussées dans un moyen de culture containent Kanemycine.

3. Vecteur plasmidique pour la clonage et l'expression de portions des chaînes d'immunoglobulines dans des plantes, selon une quelconque des revendication précédentes, dans lequel ledit promoteur comprend des éléments de régulation du gène de virus CaMV 35S (le virus à mosaique du chou-fleur).

4. Vecteur plasmidique pour la donage et l'expression de portions des chaînes d'immunoglobulines dans des plantes, selon une quelconque des revendication précédentes, dans lequel ladite séquence des détecteurs codefie un peptide, qui est capable de réagir avec un anticorps connu.

5. Vecteur plasmidique pour la clonage et l'expression de portions des chaînes d'immunoglobulines dans des plantes, selon une quelconque des revendication précédentes, dans lequel ledit terminateur est le terminateur du gène de synthètase de nopaline,

6. Vecteur plasmidique pour la clonage et l'expression de portions des chaînes d'immunoglobulines dans des plantes, selon une quelconque des revendication précédentes, dans lequel lesdites portions des chaînes d'immunoglobulines réagissent dans un manière spécifique avec une composition chimique ou avec des agents pathogènes, en conférrand à la plante transformée une ésistance à lesdites compositions chimique ou agents pathogènes.

7. Vecteur plasmidique pour la donage et l'expression de portions des chaînes d'immunoglobulines dans des plantes, selon une quelconque des revendication précédentes, dans lequel ledit vecteur comprend une séquence de codification pour un peptide de signal, qui est lié de façon covalent au terminal NH₂ des portions des chaînes d'immunoglobulin, capable de promouvoir la transportation et la sécrétion extracellulaire desdites portions des chaînes d'immunoglobuline.

8. Vecteur plasmidique pour la clonage et l'expression de portions des chaînes d'immunoglobulines dans des plantes, selon une quelconque des revendication précédentes, qui consiste du plasmid pBG-d-Ab-BIN, qui est été deposité, après la transformation of E. coli avec lemême, avec le NCAIM sous le numéro d'acces 001144 le 31.May 1991, en conformité avec le Traité de Budapest.

9. Procédé pour la transformation des plantes au moyen du vecteur plasmidique pour la donage et l'expression de portions des chaînes d'immunoglobulines dans des plantes, selon une quelconque des revendication précédentes, dans lequel ledit vector comprenant les phases suivantes:
- transformer des bactéries appartenants au Agrobacterium gène au moyen dedit vecteur pour obenir des bactéries transformés;
- infecter la plante à transformée par lesdits bactéries transformés;
- sélectionner la plant transgènique ainsi obtenue au moyen dedit marqueur sélectionnable qui est exprimé dans des plantes;
- contrôler le niveau d'expression desdites portions des chaînes d'immunoglobuline dans des plantes transgèniques au moyen du produit codifié par ladite séquence des detecteurs.

10. Plantes ou leur portions transformées au moyen des vecteurs selon une quelconque des revendication précédentes 1 à 8.

11. Portions d'une plante selon la revendiation 10, dans lesquelles desdites portions comprendent le matériel de reproduction.

12. Plantes ou leur portions selon la revendication 10 ou 11, qui peuvent être obtenues au moyen du procédé selon la revendication 9.
